# EUROPEAN PATENT APPLICATION

(11) **EP 4 197 498 A1**
(43) Date of publication of application: **21.06.2023**
(21) Application number: 22214359.6
(22) Date of filing: 16.12.2022
(51) Int. Cl.: A61F 2/95, A61F 2/966

(54) **AN ENDOGRAFT SYSTEM HAVING A DEPLOYMENT DEVICE AND A SENT-GRAFT ASSEMBLY**

(30) Priority: 17.12.2021 AU 2021286428
(71) Applicant: Cook Medical Technologies LLC, Bloomington, IN 47404 (US)
(72) Inventor: COLLINS, James, Paddington, 4064 (AU)
(74) Representative: Williams Powell

(57) **Abstract**

An endovascular system comprises: a deployment device (100) and a stent-graft assembly (140). The stent-graft assembly comprises: an elongate vessel wall engaging graft portion (145); and a proximal-end self-expanding stent (200) comprising a plurality of terminal portions (210). The deployment device (100) comprises: an elongate guide wire catheter (120) adapted to be deployed over a guide wire (7); a tip (120) at a proximal end of the guide wire catheter (120). The tip has a proximal nose and a distal receiving zone (134). The distal receiving zone comprises a plurality of circumferentially spaced apart elongate recesses (136-138), each recess receiving at least one of the terminal portions. A trigger wire arrangement (610-640) retaining the at least one terminal portions within a corresponding recess of the receiving zone is also provided. Retention of the terminal portions within the recess of the receiving zone constrains against relative twisting between the stent-graft assembly and the tip.

## Description

### TECHNICAL FIELD

The disclosure relates to medical devices and grafts, sometimes known as endografts, and more particularly to endograft systems having a deployment device and a stent-graft assembly.

### BACKGROUND

This disclosure will be particularly discussed in relation to stent-grafts for placement into the aorta for the treatment of aneurysms. The disclosure, however, is not so restricted and may be applied to stent-grafts for placement in any lumen of the human or animal body.

In the deployment of an endograft, or stent-graft, into the human or animal body via intraluminal techniques, a deployment device is used to introduce the stent-graft into a lumen of the body and, after the stent-graft has been deployed and expanded within the lumen, the deployment device needs to be retracted and removed from the body.

The introducer may include a proximal nose cone with a distally facing capsule to enclose, or partially enclose, an exposed stent of a stent-graft during introduction. After the stent-graft has been released and the capsule has been removed from the exposed stent, the capsule along with the introducer is withdrawn. The capsule, however, typically has a distally facing opening with an edge surrounding it and this edge can engage with stents of the deployed stent-graft, and potentially cause problems by dislodging the stent-graft from its position on the wall of the lumen. Even if an edge of the distally facing opening does not dislodge the stent-graft, it may catch on an internal surface of a vessel such as the aorta.

It is known to provide moveable capsule plugs to facilitate retrieval of introducers. However, known capsule assemblies comprising capsules and capsule plugs have various shortcomings, including adding complexity to both manufacture and use.

Throughout this specification, the term distal with respect to a portion of the aorta, a deployment device or an endograft means the end of the aorta, deployment device or endograft further away in the direction of blood flow away from the heart and the term proximal means the portion of the aorta, deployment device or end of the endograft nearer to the heart. When applied to other vessels, similar terms such as caudal and cranial should be understood.

### SUMMARY

According to an aspect of the disclosure there is provided a deployment device and medical device assembly,
the medical device comprising:
   an elongate vessel wall engaging portion; and
   a proximal-end self-expanding stent comprising a plurality of terminal portions, and
the deployment device comprising:
   an elongate guide wire catheter configured to be deployed over a guide wire;
   a tip at a proximal end of the guide wire catheter, the tip having a proximal nose and a distal receiving zone, the distal receiving zone comprising one or more elongate recesses, the or each elongate recess receiving at least one of the terminal portions; and
   a terminal portion retention arrangement retaining the at least one terminal portions within the or a corresponding elongate recess of the receiving zone,
wherein the retention of the terminal portions within the one ore more elongate recesses of the receiving zone constrains against relative twisting between the medical device and the tip.

The medical device could be any one of more of: a stent graft, a stent, a filter, an occlusion device, a prosthetic valve, an embolization coil, or any other medical device.

According to an aspect of the disclosure there is provided an endovascular system, the system comprising: a deployment device and a stent-graft assembly,
the stent-graft assembly including:
   an elongate vessel wall engaging graft portion; and
   a proximal-end self-expanding stent including a plurality of terminal portions, and
the deployment device including:
   an elongate guide wire catheter adapted to be deployed over a guide wire;
   a tip at a proximal end of the guide wire catheter, the tip having a proximal nose and a distal receiving zone, the distal receiving zone including one or more elongate recesses, each recess receiving at least one of the terminal portions; and
   a trigger wire arrangement retaining the at least one terminal portions within the or a corresponding recess of the receiving zone,
wherein the retention of the terminal portions within the one or more recesses of the receiving zone constrains against relative twisting between the stent-graft assembly and the tip.

The distal receiving zone preferably comprises a plurality of circumferentially spaced apart elongate recesses.

Preferably, the deployment device comprises a sheath, the sheath having an initial position covering the stent-graft assembly and a retracted condition in which the stent-graft assembly is uncovered.

Advantageously, each recess receives a group of terminal portions.

The tip preferably comprises three recesses, each of the three recesses receiving a group of terminal portions.

The proximal-end self-expanding stent may comprise terminal bends and distal bends, the terminal bends and distal bends joined by struts.

Preferably, each terminal portion comprises a terminal bend and at least a portion of each of two adjacent struts.

The terminal portions advantageously include barbs projecting from portions of struts, the barbs arranged to anchor the stent-graft assembly within a vessel wall.

The barbs within each group of terminal portions are preferably staggered with respect to each other.

In the preferred embodiments, the trigger wire arrangement comprises:
a distal release wire having a first actuation end at a handle of the deployment device and a first terminal end terminating within the tip; and
a proximal release wire having a second actuation end at the handle of the deployment device and a second terminal end terminating within the tip,
wherein the distal release wire retains distal portions of each of the groups of terminal portions, and
wherein the proximal release wire retains proximal portions of each of the groups of terminal portions.

The trigger wire arrangement may comprise a plurality of proximal release wires.

According to another aspect of the disclosure there is provided an endovascular system, the system comprising: a deployment device and a stent-graft assembly,
the stent-graft assembly comprising:
   an elongate vessel wall engaging graft portion;
   a proximal-end self-expanding stent comprising a plurality of terminal portions, and
the deployment device comprising:
   an elongate guide wire catheter adapted to be deployed over a guide wire;
   a tip at a proximal end of the guide wire catheter, the tip having a proximal nose and a distal receiving zone, the distal receiving zone comprising three circumferentially spaced apart elongate recesses, each recess receiving at least one of the terminal portions;
   a trigger wire arrangement retaining the at least one terminal portions within a corresponding recess of the receiving zone; and
   a sheath, the sheath having an initial position covering the stent-graft assembly and a retracted condition in which the stent-graft assembly is uncovered,
wherein the retention of the terminal portions within the recess of the receiving zone constrains against relative twisting between the stent-graft assembly and the tip.

Preferably, the trigger wire arrangement comprises three proximal release wires, one proximal release wire for each recess.

Advantageously, each recess receives a group of terminal portions.

The proximal-end self-expanding stent preferably comprises terminal bends and distal bends, the terminal bends and distal bends joined by struts.

Each terminal portion preferably comprises a terminal bend and at least a portion of each of two adjacent struts.

The terminal portions advantageously include barbs projecting from portions of struts, the barbs arranged to anchor the stent-graft assembly within a vessel wall.

The barbs within each group of terminal portions may be staggered with respect to each other.

Preferably, the trigger wire arrangement comprises:
a distal release wire having a first actuation end at a handle of the deployment device and a first terminal end terminating within the tip; and
each of the three proximal release wire having a second actuation end at the handle of the deployment device and a second terminal end terminating within the tip,
wherein the distal release wire retains distal portions of each of the groups of terminal portions, and
wherein the proximal release wire retains proximal portions of each of the groups of terminal portions.

### BRIEF DESCRIPTION OF THE DRAWINGS

Embodiments of the present invention are described below, by way of example only, with reference to the accompanying drawings, in which:
Figure 1A is an isometric view of a prior art endovascular system for delivering a stent-graft assembly;
Figure 1B is a diagrammatic cross-sectional view of the proximal end of the prior art endovascular system of Figure 1A;
Figure 2 is an isometric view of an endovascular system for delivering a stent-graft assembly according to the teachings herein;
Figures 3A and 3B are isometric views of the tip of the endovascular system of Figure 2;
Figures 4A to 4F are magnified side views of the proximal end of the endovascular system of Figure 2;
Figure 4G is a similar magnified side view to that of Figure 4F, but shows an arrangement having staggered barbs;
Figures 5A and 5B are isometric views of the tip, similar to that of Figures 3A and 3B, but showing a tip optimized for pre-cannulated procedures;
Figure 6 is an isometric view of the tip of Figure 5B, but in a more close up view showing the constraint of terminal portions of the proximal-end self-expanding stent;
Figures 7A and 7B are cross-sectional view towards the distal end of the tip of Figure 6;
Figures 8A and 8B are isometric views of another example of tip of the endovascular system of Figure 2, the tip having two rather than three circumferentially spaced apart elongate recesses;
Figure 9 is a cross-sectional view towards the distal end of the tip of Figures 8A and 8B;
Figures 10A and 10B are isometric views of a tip of the endovascular system of Figure 2, the tip having four rather than three circumferentially spaced apart elongate recesses;
Figure 11 is a cross-sectional view towards the distal end of the tip of Figures 10A and 10B; and
Figure 12 is isometric view of the tip similar to that of Figure 6, but shows the constraint of terminal portions of a proximal-end self-expanding stent having staggered barbs as shown in Figure 4G.

### DESCRIPTION OF PREFERRED EMBODIMENTS

The preferred embodiments described below are directed to a medical device being a stent-graft assembly. It is to be understood that this is jus tone application of the disclosure herein. The assembly can be used for the deployment of medical devices in general, such as one of more of: a stent graft, a stent, a filter, an occlusion device, a prosthetic valve, an embolization coil, or any other medical device, typically an endoluminally implantable medical device.

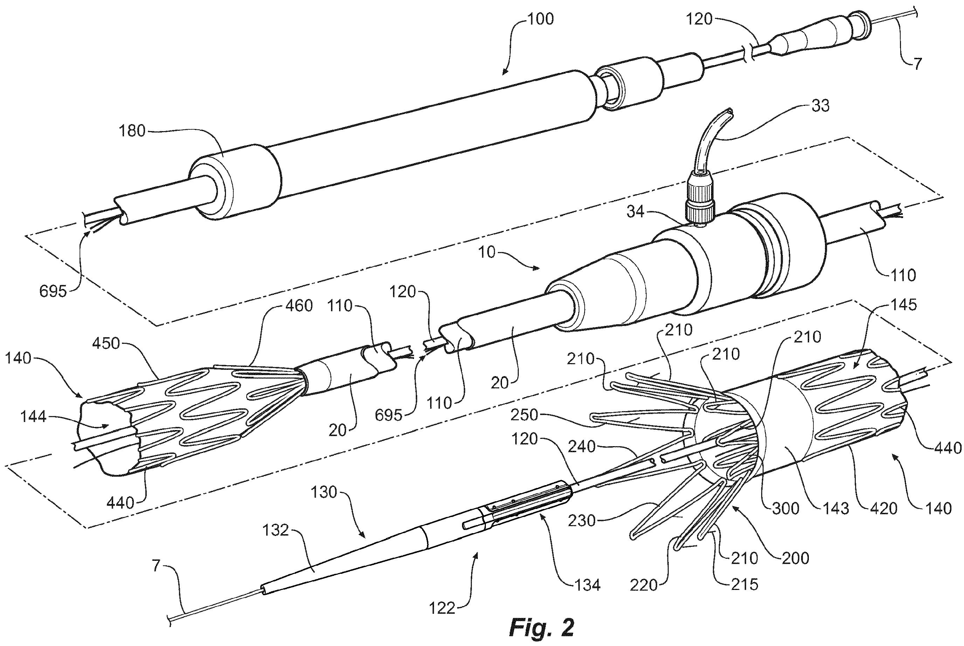

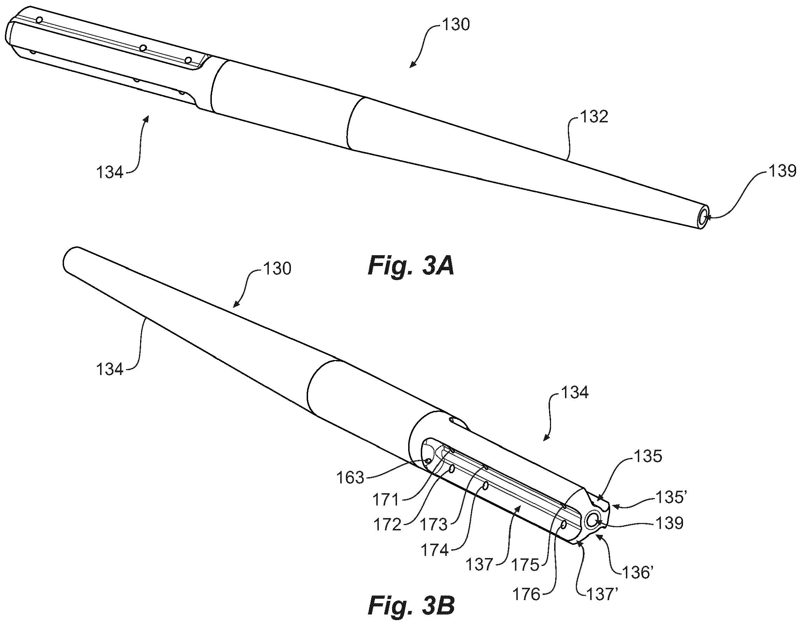

Referring to Figures 2, 3A and 3B, an endovascular system 1 according to the teachings herein is shown. The system comprises: a deployment device 100 and a stent-graft assembly 140.

As illustrated in Figure 2, the stent-graft assembly comprises: an elongate vessel wall engaging graft portion 145, the graft portion defining an inner lumen 144. The stent-graft also has a proximal-end self-expanding stent 200 comprising a plurality of terminal portions 210. In this embodiment, the proximal-end self-expanding stent is a bare stent. That is, the stent is not covered, and is not covering, the graft portion 145 to any significant degree. Instead it extends proximally and, when released, is free to expand beyond an outer diameter of the graft portion 145. This enables it to strongly engage with a vessel wall in which it is deployed.

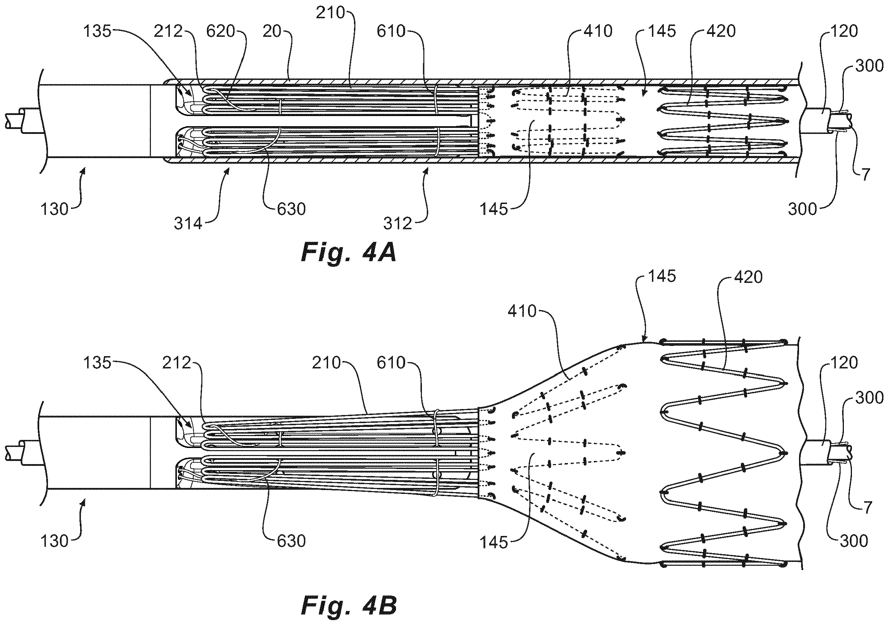

Figure 2 also shows external stents 420, 440, 450 and 460 that extend along the outside of the graft portion 145. An internal stent 410, shown in Figures 4A and 4B, is provided inside the sealing zone 143. This internal stent 410 exerts a radially outwards force to assist in effecting a seal between the sealing zone 143 and the inner wall of the vessel into which the stent-graft is deployed.

Again referring to Figure 2, it can be seen that the deployment device 100 comprises: an elongate guide wire catheter 120 adapted to be deployed over a guide wire 7. A tip 130 is located at a proximal end 122 of the guide wire catheter 120, the tip having a proximal nose 132 and a distal receiving zone 134. The distal receiving zone 134 comprising a plurality of circumferentially spaced apart elongate recesses, in the embodiment illustrated in Figure 3A and 3B, three recesses 136, 137, 138. Each of the three recess, clearly show in Figure 3B, has a distally opening end 136', 137', 138' and each recess receives at least one of the terminal portions 210 through a respective distally opening end 136', 137', 138'.

The deployment device 100 also comprises a sheath 20 which can be seen in Figures 2 and 4A. The sheath 20 has an initial position covering the stent-graft assembly 140, as shown in Figure 4A, and a retracted condition in which the stent-graft assembly 140 is uncovered, as shown in Figure 4B. The sheath 30 is part of the introducer 10 as shown in Figure 2. The introducer 10 includes a flushing tube 33 and a port 34 for flushing.

Figures 3A and 3B show the tip 130 in detail, separate from the remainder of the deployment device 100. The tip catheter 139 that forms part of the guide wire catheter 120 allows for the passage of the guide wire 7 over which the entire deployment device slides. For example, the guide wire may extend from an incision in the femoral artery up into the thoracic aorta when the endovascular system 1 is used to deploy the stent-graft assembly 140 to repair a TAA (Thoracic Aortic Aneurysm).

Typically the stent-graft assembly 140 will be deployed into the femoral artery using the Seldinger technique. This technique involves creating a surgical opening in the vascular system linked to the vessel of interest with a needle and inserting a wire guide into the vessel through a bore of the needle. For example, the femoral artery may be used to access the aorta. The needle can be withdrawn, leaving the wire guide in place. A deployment device, such as the deployment device 100 shown in Figure 2, is then inserted over the wire guide and into the vessel. In Figure 2, a guide wire 7 is shown over which a tip 130 slides.

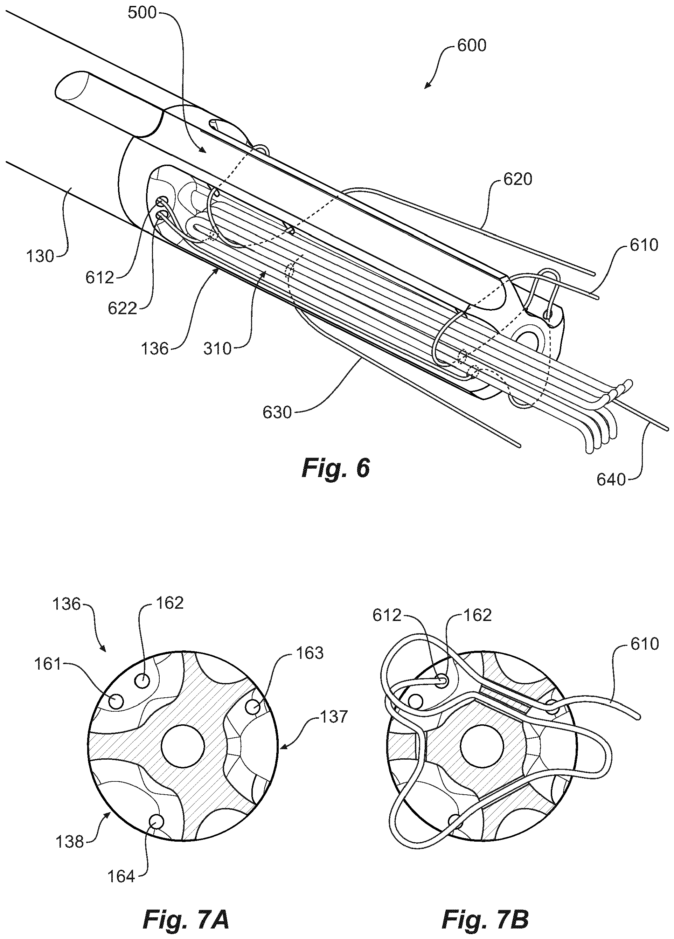

Referring now to Figures 6 and 7B, holes 171, 172, 173, 174, 175, 176 within the tip 130 are provided to receive proximal and distal release wires as is described in detail below. Blind holes 161, 162, 163 and 164 are also provided, as can be seen most clearly in Figure 7A. These blind holes receive terminal ends 612 and position these ends safely.

The deployment device 100 includes a trigger wire arrangement retaining the at least one terminal portions 210 within a corresponding recess of the receiving zone. The position in which the terminal portions 210 are held is most clearly shown in Figures 4A and 4B. The retention of the terminal portions 210 within the recess 136, 137, 138 of the receiving zone 134 constrains against relative twisting between the stent-graft assembly 140 and the tip 130.

Embodiments of the invention as described constrain against twisting. This is important, for instance during aortic intervention, where it is desirable to set the rotational position of the stent-graft assembly 140 relative to the aorta and its branch vessels, such as the renal arteries, optimally.

Now referring to Figure 6, it can be seen that each recess receives a group of terminal portions 310. Figure 6 only shows one of three groups of terminal portions 310 within recesses 136 for clarity (the remaining two terminal portions are received within the other two recesses 137 and 138). In Figures 4A and 4B, two of the three recesses, together with terminal portions 210, are shown.

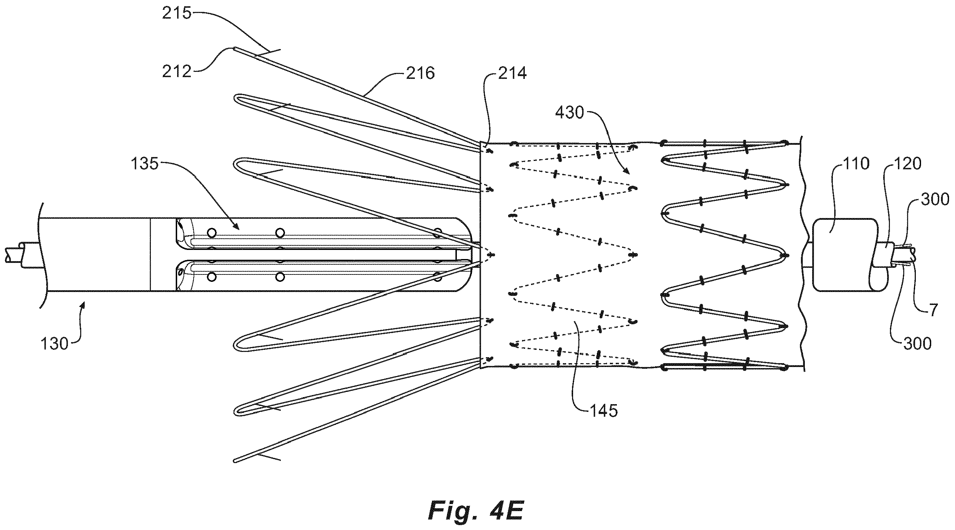

Referring to Figure 4E, it can be seen that the proximal-end self-expanding stent 200 comprises terminal bends 212 and distal bends 214, and terminal bends and distal bends joined by struts 216. Each terminal portion comprises a terminal bend 212 and at least a portion of each of two adjacent struts.

The terminal portions include barbs 250 projecting from portions of struts. The barbs 250 are provided and arranged to anchor the stent-graft assembly 140 within a vessel wall, such as a major artery, including the aorta.

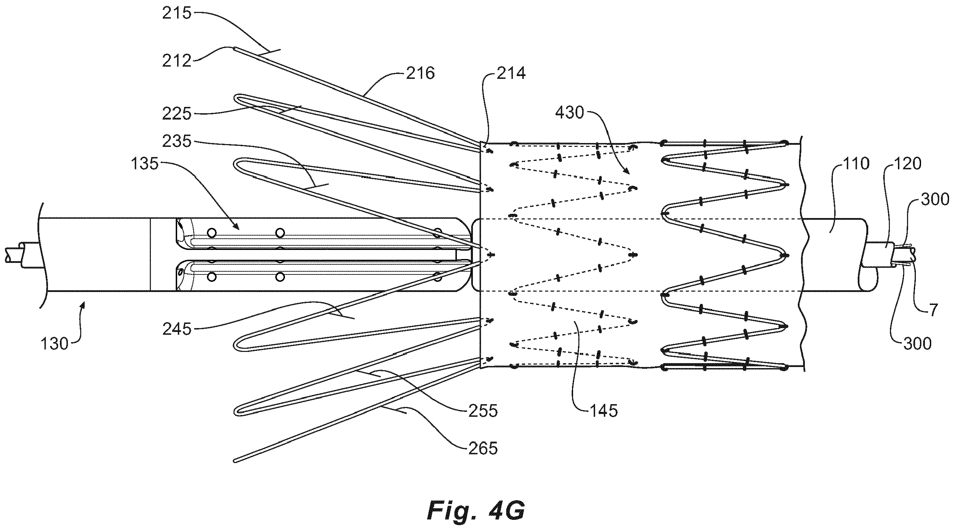

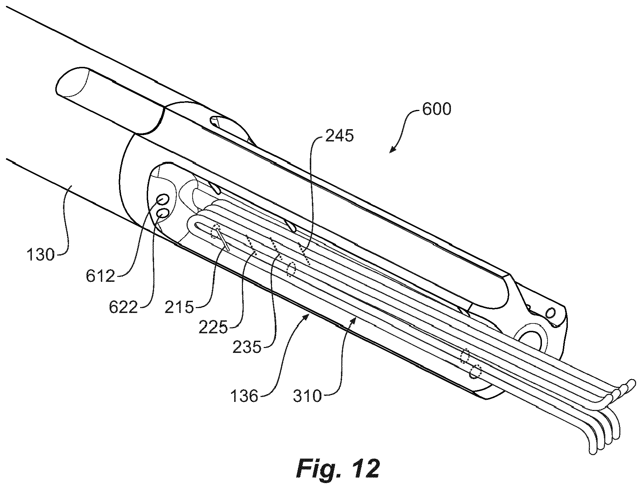

In some versions of the device, the barbs 250 within each group of terminal portions 310 are staggered with respect to each other, as is shown in Figure 4G. This allows for a very compact arrangement where the barbs 215, 225, 235 do not interfere with each other or add bulk, as is illustrated in Figure 12.

The trigger wire arrangement is most clearly shown in Figures 4A to 4F, 6, 7A and 7B. It includes: a distal release wire 610 having a first actuation end at a handle 180 of the deployment device 100 (the handle 180 shown in Figure 2) and a first terminal end 612 terminating within the tip (the tip shown in Figures 6, 7A and 7B). The distal release wire 610 retains distal portions 312 (shown in Figure 4A) of each of the groups of terminal portions.

The trigger wire arrangement also includes three proximal release wires 620, 630, 640 having a second actuation end at the handle 180 of the deployment device 100 and second terminal ends 622, 632, 642 terminating within the tip 130. The proximal release wires 620, 630 and 640 retain the proximal portions 314 (shown in Figure 4A) of each of the groups of terminal portions.

It has been found that three wires work well, however in other embodiments, fewer or more wires may be used. The number of release wires is an optimization of simplicity as compared to pull force. More wires add complexity but can lower the pulling force required to release the proximal end.

The routing of the release wires 610, 620, 630, 640 through the deployment device 100 is shown in Figure 2. In the cutaway portions of the illustration of the deployment device 100 of Figure 2 these wires 695 are shown.. The proximal ends of those wires are not shown in Figure 2, but they are shown in the more magnified views of Figures 4A to 4E.

Progressive and controlled release of the stent-graft assembly 140 will now be described with reference to Figures 4A to 4F.

Referring first to Figure 4A, a proximal portion of the endovascular system 1 is shown in a cross-sectional view. Figure 4A shows the relative location of the parts of the endovascular system 1 in their initial position. It is in the position illustrated that the deployment device 100 is inserted into the vascular system of a patient. For example, during aortic intervention, the deployment device 100 may extend up through the femoral artery into the aorta. As can be seen in Figure 4A, the sheath 20 extends over the entire stent-graft assembly 140. The proximal end of the sheath 20 extends over the entire receiving zone 134 of the tip 130. The sheath 20 is retracted, or the tip 130 is extended, so as to deploy the stent-graft assembly 140. The stent-graft assembly 140 is then exposed, or partially exposed, as is shown in Figure 4B. At this time, it can be seen that the terminal portions 210 of the proximal end of the now expanded proximal-end self-expanding stent 200 are constrained against relative twisting between the graft assembly 140 and the tip 130.

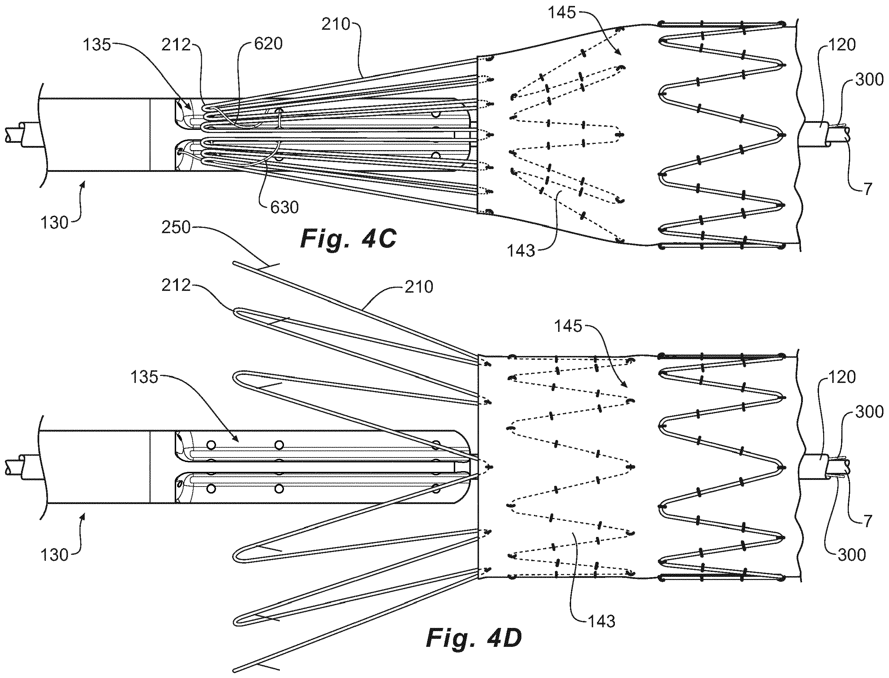

Referring now to the progression from the position shown in Figure 4B to the position shown in Figure 4C, the surgeon retracts the distal release wire 610 using an actuator on the handle 180 which allows the stent-graft assembly 140 to expand partially to the position shown in Figure 4C. In this position, good control over the final positioning of the proximal end of the stent-graft assembly 140 is still possible since the proximal end of the stent-graft 140 is held to the tip 130 by the proximal release wires 620, 630 and 640.

Once the surgeon is satisfied with the positioning of stent-graft 140 within the anatomy of the patient, the proximal release wires 620, 630 and 640 may be retracted by actuation at the handle 180 so that the proximal-end self-expanding stent 200 expands to the position shown in Figure 4D. In this position, the barbs 250 engage into the wall of the vessel into which the stent-graft is being deployed. These barbs 250 mitigate against distal movement of the graft 140 relative to the vessel wall.

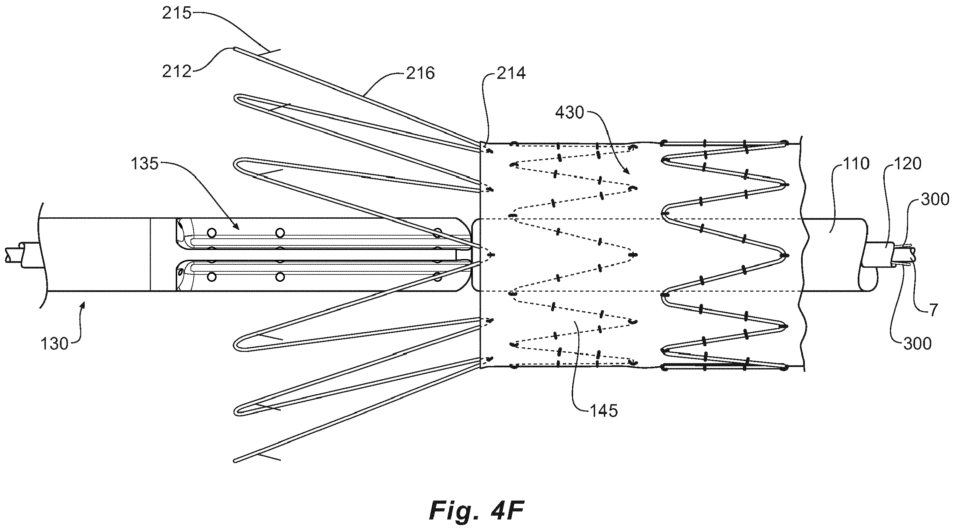

Referring now to Figures 4E and 4F, the process of withdrawal of the deployment device 100 is shown. In Figure 4E, it can be seen that the pusher 110 has been advanced towards the tip 130. In Figure 4F, it can be seen that the pusher 110 has advanced further to abut the distal end of the tip 130. In the position shown in Figure 4F, the deployment device 100 is able to be fully withdrawn through the stent-graft assembly 140.

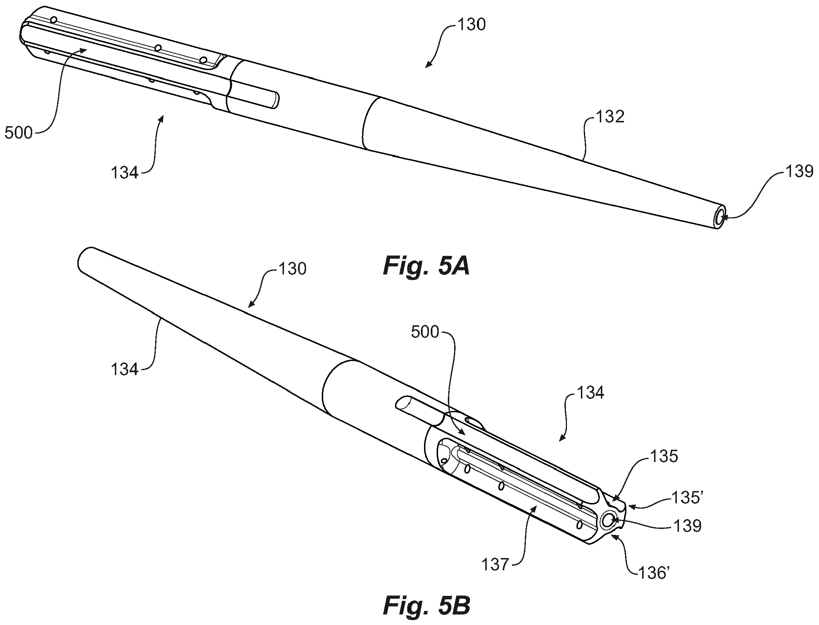

Referring to Figures 5A, 5B and 6, a tip 130 having an additional groove 500 is shown, extending longitudinally along the tip 130 and in an outer surface thereof. This is a groove to accommodate a small branch cannula, such as a renal cannula. This allows the endovascular system 1 to be used in pre-cannulated systems, in which case the groove 500 may be better described as a pre-load groove 500

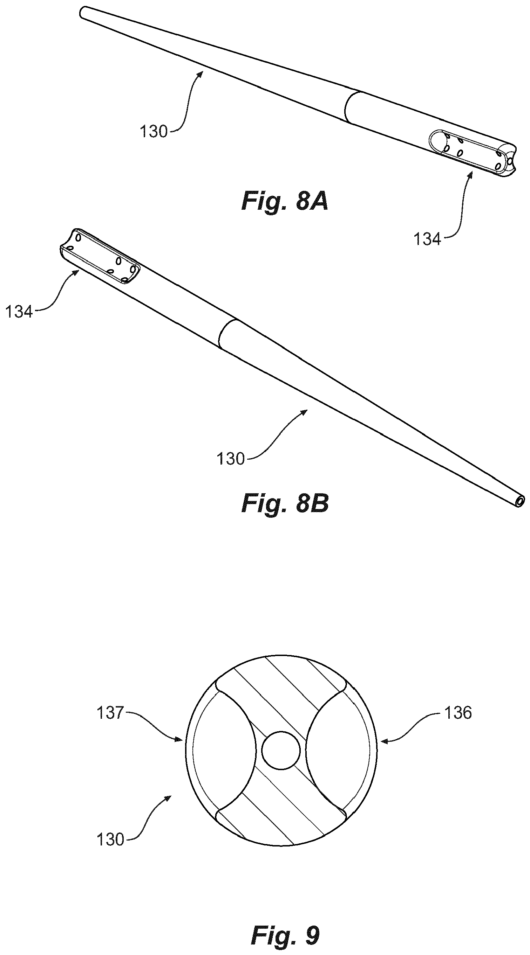

Figures 8A and 8B show isometric views of another example of tip 130 of the endovascular system 1 of Figure 2. The tip 130 has two rather than three circumferentially spaced apart elongate recesses, the two recesses being recesses 136, 137 as is most clearly show in Figure 9, a cross-sectional view towards the distal end of the tip 130 of Figures 8A and 8B.

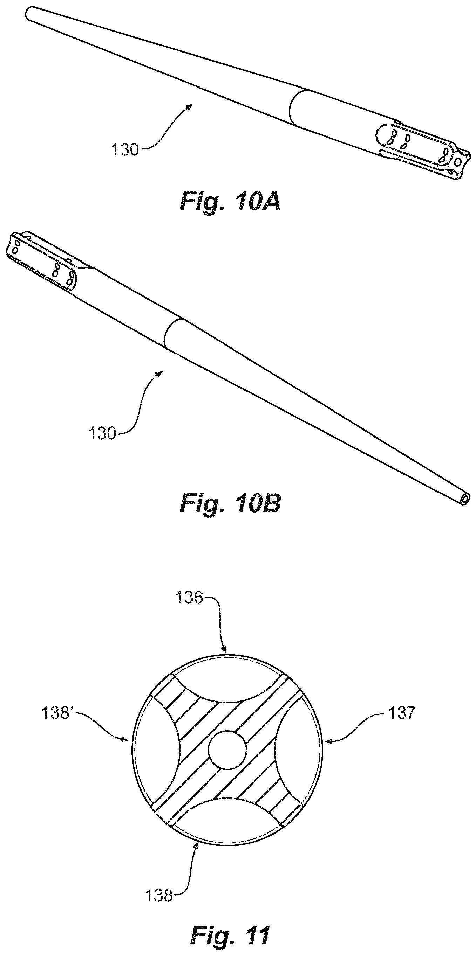

Figures 10A and 10B are isometric views of another example of tip 130 of the endovascular system 1 of Figure 2, the tip having four rather than three circumferentially spaced apart elongate recesses, the four recesses being recesses 136, 137, 138, 139 as is most clearly show in Figure 11, a cross-sectional view towards the distal end of the tip 130 of Figures 10A and 10B.

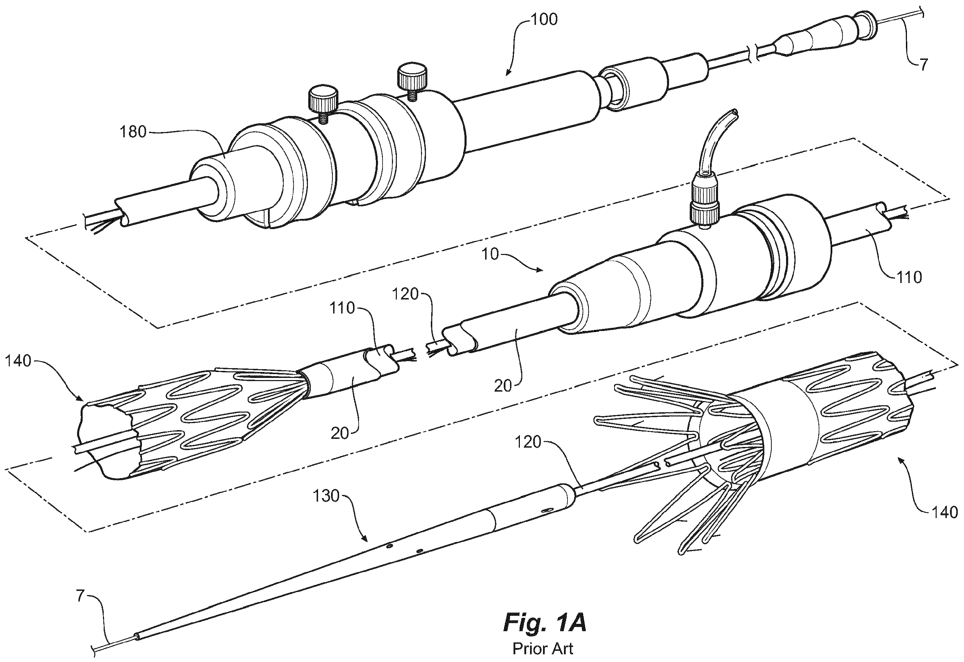

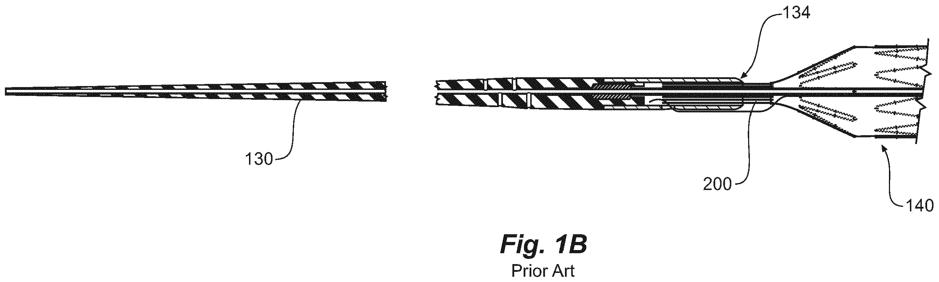

Figures 5A, 5B and 6 also show that the distal receiving zone 134 comprises three circumferentially spaced apart elongate recesses 136, 137, 138. This arrangement is an improvement on the prior art arrangements, including the prior art arrangement shown in Figures 1A and 1B. In particular, the prior art receiving zone 134 as shown in Figure 1B, for receiving barbed proximal-end self-expanding stent 200 at the proximal end of the stent-graft 140, presents a distally opening mouth. This mouth and any edge surrounding presents a risk that it may dislodge the stent-graft from its position on the wall of the lumen. It could also damage arterial or other vessels. While solutions to that problem such as dockable top cap mechanisms exist, they have disadvantages, including adding complexity to the deployment device 100. In contrast the constraint arrangement formed by the elongate recesses 136, 137, 138 and the trigger wire arrangement described above, offers a very simple and reliable solution. It also greatly decreases the probability of the stent-graft 140 twisting relative to the deployment device 100, decreases the risk of entanglement during release and only uses one deployment wire. Finally, it also improves the stent-graft position visibility within the sheath.

Throughout this specification various indications have been given as to the scope of this invention but the invention is not limited to any one of these but may reside in two or more of these combined together. The examples are given for illustration only and not for limitation.

Throughout this specification and the claims that follow unless the context requires otherwise, the words 'comprise' and 'include' and variations such as 'comprising' and 'including' will be understood to imply the inclusion of a stated integer or group of integers but not the exclusion of any other integer or group of integers.

The disclosures in Australian Patent Application No. 2021/286428, from which this application claims priority, and in the abstract accompanying this applciaiton are incorporated herein by reference.

## Claims

1. A deployment device and medical device assembly,
the medical device comprising:
an elongate vessel wall engaging portion; and
a proximal-end self-expanding stent comprising a plurality of terminal portions, and
the deployment device comprising:
an elongate guide wire catheter configured to be deployed over a guide wire;
a tip at a proximal end of the guide wire catheter, the tip having a proximal nose and a distal receiving zone, the distal receiving zone comprising one or more elongate recesses, the or each elongate recess receiving at least one of the terminal portions; and
a terminal portion retention arrangement retaining the at least one terminal portions within the or a corresponding elongate recess of the receiving zone,
wherein the retention of the terminal portions within the one ore more elongate recesses of the receiving zone constrains against relative twisting between the medical device and the tip.

2. A system according to claim 1, comprising:
a trigger wire arrangement retaining the at least one terminal portions within a corresponding elongate recess of the receiving zone.

3. A system according to claim 1 or 2, wherein the medical device is a stent-graft assembly,
the stent-graft assembly comprising:
an elongate vessel wall engaging graft portion; and
a proximal-end self-expanding stent comprising a plurality of terminal portions;
wherein the retention of the terminal portions within the elongate recess of the receiving zone constrains against relative twisting between the stent-graft assembly and the tip.

4. The endovascular system of any preceding claim, wherein the deployment device comprises a sheath, the sheath having an initial position covering the medical device assembly and a retracted condition in which the medical device is uncovered.

5. The endovascular system of any preceding claim, wherein each elongate recess is sized to receive a group of terminal portions.

6. The endovascular system of any preceding claim, wherein the proximal-end self-expanding stent comprises terminal bends and distal bends, the terminal bends and distal bends joined by struts.

7. The endovascular system of claim 6, wherein each terminal portion comprises a terminal bend and at least a portion of each of two adjacent struts.

8. The endovascular system of any preceding claim, wherein the terminal portions include barbs projecting from portions of struts, the barbs arranged to anchor the medical device within a vessel wall.

9. The endovascular system of claim 8, wherein the barbs within each group of terminal portions are staggered with respect to each other.

10. The endovascular system of any preceding claim, wherein the or a trigger wire arrangement comprises:
a distal release wire having a first actuation end at a handle of the deployment device and a first terminal end terminating within the tip; and
a proximal release wire having a second actuation end at the handle of the deployment device and a second terminal end terminating within the tip,
wherein the distal release wire retains distal portions of each of the groups of terminal portions, and
wherein the proximal release wire retains proximal portions of each of the groups of terminal portions.

11. The endovascular system of claim 10, wherein the trigger wire arrangement comprises a plurality of proximal release wires.

12. The endovascular system of any preceding claim, wherein each elongate recess receives a group of terminal portions.

13. The endovascular system of any preceding claim, wherein the tip comprises three elongate recesses, each of the three elongate recesses receiving a group of terminal portions.

14. The endovascular system of claim 13, wherein the trigger wire arrangement comprises three proximal release wires, one proximal release wire for each elongate recess.

15. The endovascular system of claim 13 or 14, wherein the trigger wire arrangement comprises:
a distal release wire having a first actuation end at a handle of the deployment device and a first terminal end terminating within the tip; and
each of the three proximal release wire having a second actuation end at the handle of the deployment device and a second terminal end terminating within the tip,
wherein the distal release wire retains distal portions of each of the groups of terminal portions, and
wherein the proximal release wire retains proximal portions of each of the groups of terminal portions.
